# EUROPÄISCHE PATENTSCHRIFT

(11) **EP 1 066 019 B1**
(45) Veröffentlichungstag und Bekanntmachung des Hinweises auf die Patenterteilung: **05.04.2006**
(21) Anmeldenummer: 00904933.9
(22) Anmeldetag: 19.01.2000
(51) Int. Cl.: A61K 8/49, A61Q 5/04, A61K 8/37, A61K 8/81

(54) **VERFAHREN ZUR DAUERHAFTEN HAARVERFORMUNG MIT ZEITABHÄNGIGER REDUZIERUNG DER WELLWIRKSAMKEIT**
METHOD FOR PERMANENTLY SHAPING HAIR WITH TIME-DEPENDENT REDUCTION OF THE WAVE EFFECT
PROCEDE DE PERMANENTE AVEC REDUCTION DE L'EFFET D'ONDULATION DES CHEVEUX EN FONCTION DU TEMPS

(30) Priorität: 21.01.1999 DE 19902246
(43) Veröffentlichungstag der Anmeldung: 10.01.2001
(73) Patentinhaber: Wella Aktiengesellschaft, 64274 Darmstadt (DE)
(72) Erfinder: REGENBOGEN, Rainer, CH-1723 Marly (CH); MARESCH, Gerhard, D-64295 Darmstadt (DE); LANG, Günther, D-64354 Reinheim (DE); BITTERLI, Natalie, CH-3280 Murten (CH)
(86) Internationale Anmeldenummer: PCT/EP2000/000387
(87) Internationale Veröffentlichungsnummer: WO 2000/042978

(56) Entgegenhaltungen:
- DE-A- 2 349 050
- DE-A- 2 421 248
- DE-A- 3 534 287
- US-A- 3 966 903
- US-A- 4 666 712

## Beschreibung

Die vorliegende Erfindung betrifft ein Verfahren zur dauerhaften Verformung von Haaren auf der Basis eines keratinreduzierenden Wirkstoffs und einer alkalisch hydrolisierbaren Verbindung unter allmählichen Reduzierung der Wellwirksamkeit und schonender Verformung von Haaren mit hoher Ergebnissicherheit und verbesserten Pflegeeigenschaften.

Der Vorgang der dauerhaften Haarumformung besteht im allgemeinen aus zwei Schritten. Zunächst werden die Cystin-Disulfidbrücken des Haarkeratins mit Hilfe eines Mittels, das einen keratinreduzierenden Wirkstoff enthält, geöffnet. Eine Umformung - entweder Wellen von glattem Haar oder Glätten von krausem Haar - ist in dieser Phase möglich, indem im Anschluß an die Reduktion das Haar gespült und die Cystin-Disulfidbrücken unter Verwendung eines einen oxidierenden Wirkstoff enthaltenden Mittels in ihrer neuen Position wiederhergestellt werden.

Als reduzierender Wirkstoffe werden hierbei Mercaptocarbonsäuren, insbesondere Thioglykolsäure oder Thiomilchsäure, beziehungsweise deren Salze, Mercaptocarbonsäureester, Cystein und dessen Derivate oder Cysteamin und dessen Derivate eingesetzt.

Die Thioglykolat enthaltenden Zusammensetzungen werden üblicherweise bei einem pH-Wert zwischen 8 und 10, insbesondere 8,5 bis 9,5, eingesetzt, was bei wiederholter, zeitlich nahe zusammenliegender Anwendungen zu Haarschädigungen führen kann.

Dem hohen pH-Wert kommt hierbei, neben der verstärkten Quellung des Haares, insbesondere die für die Spaltung der Disulfidbrücken des Cystins notwendige Steigerung der Reduktionskraft der eingesetzten Mercaptoverbindung, durch erhöhte Dissoziation zu. Zur Einstellung des pH-Wertes kommen neben üblichen Alkalisierungsmitteln bevorzugt Ammoniak, Ammoniumhydrogencarbonat, Ammoniumcarbonat, Ammoniumcarbamat und Monoethanolamin zum Einsatz.

Die vorstehend genannten mildalkalischen Dauerwellpräparate werden in üblicher Anwendungsweise auf das Haar aufgetragen. Zum Erreichen einer permanenten Haarumformung muß das Mittel eine bestimmte Zeitdauer einwirken. Je nach Zustand des behandelten Haares stellt eine Verlängerung der Einwirkungszeit bzw. eine wiederholte Anwendung der Dauerwellpräparate eine zunehmende Beanspruchung im Sinne einer irreversiblen Schädigung der Haarstruktur dar. Als besondere Erschwernis kommt hinzu, daß der Reduktionsvorgang und die damit verbundene Haarerweichung auch nach Erreichen der vorgeschriebenen Einwirkzeit voranschreitet, so daß es für den Friseur sehr wichtig ist, den richtigen Endpunkt festzulegen. Besonders relevant zeigt sich dies bei langem, bereits mehrfach mit Dauerwellmitteln, oxidativen Färbemitteln und/oder Blondiermitteln vorbehandelten Haaren. Da die genannten Behandlungsmethoden die Haarstruktur beeinträchtigen, geschädigtes Haar jedoch für das Verformungsmittel besonders permeabel ist, kann bereits bei relativ geringer Überschreitung der optimalen Einwirkungszeit eine Überkrausung eintreten.

Dieses in der täglichen Friseurpraxis immer wieder auftretende Problem hat zu einer Reihe von Lösungsversuchen geführt, die der Gefahr einer Haarschädigung beim Dauenrvellprazeß, durch eine Abnahme der Wellwirksamkeit des Reduktionsmittels während der Einwirkungszeit, begegnen sollen.

In der EP-A 0 107 159 und US-A 4 666 712 werden haarkosmetische Mittel beschrieben, die zur Verbesserung der Haarstruktur und der Widerstandsfähigkeit der Haare On-Säuren, die von Aldohexosen oder Disacchariden mit unverschlossener Aldehydfunktion abgeleitet sind und/oder deren gamma- oder delta-Lactonen enthalten. Für Haarkosmetische Mittel, die starke Reduktionsmittel enthalten, werden nur die freien On-Säuren empfohlen; ebenfalls sind in den Beispielen nur freie On-Säuren eingesetzt. Die zeitliche Änderung des pH-Wertes wird dort nicht angesprochen.

Aus der eigenen DE-A 23 49 050 ist ein Mittel und ein Verfahren bekannt, bei dem die Wellaktivität über das Absenken des Anfangs-pH-Wertes kontinuierlich verringert wird. Dem Verformungsmittel werden kurz vor dem Gebrauch solche alkalisch spaltbaren organischen Verbindungen mit Ester- oder Halogengruppen im Molekül zugesetzt, die imstande sind, bei der Spaltung Säure entstehen zu lassen. Dadurch soll die Konzentration an Alkali während der Einwirkungszeit allmählich und in dem gewünschten Umfang abnehmen. Als geeignete Ester sind u. a. Essigsäureethylester und Triacetin genannt. Bei den Estergruppen enthaltenden Verbindungen kann eine Beschleunigung der Spaltung durch zusätzliche Beigabe der Lipase Pankreatin erreicht werden.

Nachteile dieses Vorschlages sind, neben der ungünstigen Toxikologie und Hautverträglichkeit vieler der erwähnten Substanzen, insbesondere die niedrigen Verseifungsgeschwindigkeiten der dort empfohlenen Ester, die dazu führt, daß ohne den Einsatz des in der Patentschrift erwähnten Enzyms Pankreatin während der zur Verfügung stehenden Einwirkungszeit von 5 bis 30 Minuten eine unzureichende Menge an Säure freigesetzt wird. Beim Einsatz von esterspaltenden Enzymen sind zudem Stabilitätsprobleme zu überwinden, die es u. a. erforderlich machen, das Verformungsmittel in drei Komponenten zu formulieren.

Der Erfindung liegt daher die Aufgabe zugrunde, ein Verfahren zur dauerhaften Verformung von Haaren zur Verfügung zu stellen, bei dem ohne die Verwendung von esterspaltenden Enzymen der pH-Wert im Verlauf der Einwirkungszeit genügend rasch sinkt, damit die Reduktionskraft des Wellmittels während der Einwirkungszeit geschwächt und dadurch die Haarstruktur geschont wird, wobei selbstverständlich dennoch eine ausreichende Umformung der Haare erreicht werden soll.

Es wurde nunmehr überraschend gefunden, daß sich die vorstehend genannten Nachteile der Verfahren des Standes der Technik vermeiden lassen, falls hierbei nach vorliegender Erfindung verfahren wird.

Gegenstand der Erfindung ist ein Verfahren zur dauerhaften Verformung von Haaren, bei dem man unmittelbar (10 Sekunden bis 3 Minuten) vor der Anwendung einem alkalischen Dauerverformungsmittel mit einem Gehalt an einem keratinreduzierenden Wirkstoff und einem anfänglichen pH-Wert von 7,5 bis 10 eine alkalisch hydrolisierbare Verbindung zugesetzt, das Haar bevor und/oder nachdem man es in der gewünschten Form festhält, mit dem gebrauchsfertigen Mittel behandelt, nach der Einwirkungszeit das Haar mit Wasser spült, oxidativ nachbehandelt, erneut mit Wasser spült, gegebenenfalls zur Wasserwelle legt, und sodann trocknet, dadurch gekennzeichnet, daß die alkalisch hydrolisierbare Verbindung so ausgewählt wird, daß der anfängliche pH-Wert ohne den Zusatz eines esterspaltenden Enzyms innerhalb von 5 bis 30 Minuten um mindestens 0,5 Einheiten herabgesetzt wird; und die

Bevorzugt liegt der anfängliche pH-Wert des alkalischen Dauerverformungsmittels bei 8 bis 9,5 und wird innerhalb von 10 bis 30 Minuten um 0,8 bis 2,0 Einheiten abgesenkt. Besonders bevorzugt wird der anfängliche pH-Wert des Dauerverformungsmittels innerhalb von 7 bis 20 Minuten um mindestens eine Einheit auf einen pH-Wert zwischen 6,5 bis 7,3 herabgesetzt. Die Anwendungstemperatur beträgt vorzugsweise 20 bis 45 °C, besonders bevorzugt 30 bis 40 °C.

alkalisch hydrolisierbare Verbindung ausgewählt ist aus den Gruppen: Lacton einer Onsäure oder einer Uronsäure, Acylsalicylsäure mit 1 bis 5 Kohlenstoffatomen in der Acylgruppe, und polymere Anhydride.

Beispiele für geeignete hydrolisierbare Verbindungen aus den vorstehenden Gruppen sind Gluconsäure-1,5-lacton, Glucuronsäure-6,3-lacton, Ribonsäure-1,4-lacton, Galactonsäure-1,4-lacton, Gulonsäure-1,4-lacton, Mannonsäure-1,4-lacton, 2-Acetoxybenzoesäure und Methylvinylether/Maleinsäureanhydrid Copolymer

Als besonders zweckmäßig im Sinne der Erfindung hat sich die Verwendung von Lactonen aus der Verbindungsklasse der On- oder Uronsäuren als alkalisch hydrolisierbare Verbindungen gezeigt. Bevorzugt werden hierbei die Lactone der On- oder Uronsäuren der Aldohexosen. Unter diesen wiederum sind ganz besonders bevorzugt Gluconsäure-1,5-lacton und Glucuronsäure-6,3-lacton.

Für die Ausführung des erfindungsgemäßen Verfahrens hat es sich als besonders zweckmäßig erwiesen, die alkalisch hydrolisierbare Verbindung in einer Konzentration von 0,5 bis 15 Gew.-%, bevorzugt 1 bis 10 Gew.-%, bezogen auf das gebrauchsfertige Dauerverformungsmittel, zu verwenden.

Die alkalisch hydrolisierbare Verbindung kann hierbei entweder in reiner Form vorliegen oder als Mischung, zusammen mit inerten, üblicherweise in Kosmetika Verwendung findenden Zusätzen wie Parfümölen, Netzmitteln, Lösungsvermittlern, Farbstoffen etc. formuliert sein. Bevorzugt liegt die alkalisch hydrolisierbare Verbindung gelöst in einem nicht wäßrigen Lösungsmittel vor, wobei das nicht wäßrige Lösungsmittel bevorzugt ein mehrwertiger Alkohol, insbesondere Glycerin, ist.

Durch geeignete Auswahl der Konzentration der Puffer Ammoniumhydrogencarbonat, Ammoniumcarbonat und/oder Ammoniumcarbamat, des pH-Wertes des Dauervertormungsmittels und der Menge der zuzufügenden alkalisch hydrolisierbaren Verbindung läßt sich so über die Abnahme der Alkaütät der Dissoziationsgrad des Reduktionsmittels und damit die Wellwirksamkeit während der Einwirkungszeit in gewünschtem Maße steuern. Vorzugsweise wird dem alkalischen Dauerverformungsmittel unmittelbar vor der Anwendung zusätzlich ein Puffer zugesetzt, dessen Wirkungsbereich bei pH 6,8 bis 7,5 liegt. Die Substanzen Ammoniumhydrogencarbonat, Ammoniumcarbonat und Ammoniumcarbamat haben, neben der Erhöhung der Wellstärke, noch die Aufgabe, den pH-Wert zum Ende der Einwirkungszeit im Bereich zwischen 6 und 7 zu puffern. Die Einsatzmenge des Puffers beträgt im allgemeinen 0,1 bis 15 Gew.%, bevorzugt 1 bis 8 Gew.%, bezogen auf das gebrauchsfertige Mittel zur dauerhaften Verformung von Haaren.

Die alkalische Hydrolyse der eingesetzten Verbindung läßt sich durch die üblicherweise bei Dauerwellbehandlungen zum Einsatz gelangende Wärmezufuhr beschleunigen.

Als keratinreduzierender Wirkstoff können insbesondere Thioglykolsäure, Thioglykolsäureamide, Thioglycerin, Thiomilchsäure, 3-Mercaptopropionsäure, Cystein, Cysteamin, Homocystein, Alkyl- oder Acylcysteine oder die Salze dieser Verbindungen, insbesondere Ammoniumthioglykolat, verwendet werden. Der keratinreduzierende Wirkstoff ist in dem gebrauchsfertigen Mittel zur dauerhaften Haarverformung in einer Menge von 1 bis 25 Gew.-%, vorzugsweise 5 bis 15 Gew.-%, enthalten.

Selbstverständlich kann das gebrauchsfertige Mittel zur dauerhaften Verformung alle für derartige Mittel üblichen und bekannten Zusatzstoffe, z. B. Verdickungsmittel, wie beispielsweise, Bentonit, Fettsäuren, Stärke, Polyacrylsäure und deren Derivate, Cellulosederivate, Alginate, Vaseline, Paraffinöle, Netzmittel oder Emulgatoren aus den Klassen der anionischen, kationischen, amphoteren oder nichtionogenen oberflächenaktiven Substanzen, beispielsweise Fettalkoholsulfate, Fettalkoholethersulfate, Alkylsulfonate, Alkylbenzolsulfate, quaternäre Ammoniumsalze, Alkylbetaine, oxethylierte Alkylphenole, Fettsäureälkanolamide oder oxethylierte Fettsäureester, ferner Trübungsmittel, wie z. B. Polyethylenglykolester, Alkohole, wie z. B.; Alkohole, wie zum Beispiel Ethanol, Propanol, Isopropanol, Polyole wie zum Beispiel Ethylenglykol, 1,2- oder 1;3-Propandiol, 1,2-, 1,3- oder 1,4-Butandiol, 1,2-, 1,3-, 1,4- oder 1,5-Pentandiol und Glycerin; Zucker wie z. B. D-Glucose, Lösungsvermittler, Stabilisatoren, Puffersubstanzen, Parfümöle, Farbstoffe sowie haarkonditionierende und haarpflegende Bestandteile, wie z. B. kationische Polymere, Lanolinderivate, Cholesterin, Pantothensäure und Betain, enthalten.

Die erwähnten Bestandteile werden in dem gebrauchsfertigen Mittel zur dauerhaften Verformung in den für solche Zwecke üblichen Mengen verwendet, z. B. die Netzmittel und Emulgatoren in Konzentrationen von insgesamt 0,2 bis 30 Gew.-%, die Alkohole in einer Menge von insgesamt 0,1 bis 20 Gew.-%, die Trübungsmittel, Parfümöle und Farbstoffe in einer Menge von jeweils 0,01 bis 1 Gew.-%, die Puffersubstanzen in einer Menge von insgesamt 0,1 bis 10 Gew.-%, Zucker, Lösungsvermittler, Stabilisatoren, sowie haarkonditionierende und haarpflegende Bestandteile in einer Menge von jeweils 0,1 bis 5 Gew.-%, während die Verdickungsmittel und Lösungsvermittler in einer Menge von insgesamt 0,5 bis 20 Gew.-% in diesem Mittel enthalten sein können.

Weiterhin können in diesem Mittel zur Wirkungssteigerung sogenannte Quell- und Penetrationsstoffe, wie z. B. Dipropylenglykolmonomethylether, 2-Pyrrolidon oder Imidazolidin-2-on, in einer Menge von 1 bis 30 Gew.-% enthalten sein.

Das gebrauchsfertige Mittel zur dauerhaften Verformung von Haaren wird erhalten durch Vermischen mehrerer Komponenten. Am günstigsten ist es, wenn das gebrauchsfertige Mittel unmittelbar (10 Sekunden bis 3 Minuten) vor der Anwendung durch Vermischen von zwei Komponenten hergestellt wird, wobei die Komponente A den keratinreduzierenden Wirkstoff und die Komponente B die alkalisch hydrolisierbare Verbindung enthält. Die einzelnen Komponenten können dabei sowohl in fester als auch in flüssiger oder verdickten Form vorliegen. Das Mittel ist dabei vorteilhaft als 2-Komponentenverpackung konfektioniert.

In einer bevorzugten Ausführungsform der erfindungsgemäßen Verfahrens zur dauerhaften Haarverformung werden die Haare wie folgt behandelt: Man gibt die alkalisch hydrolisierbare Verbindung der Komponente B vor dem Auftragen zu einer Lösung der Komponente A, welche die Mercaptoverbindung enthält. Die gewickelten Haare werden nun in bekannter Weise mit dem Verformungsmittel benetzt. Der ursprüngliche pH-Wert der Mischung von 7,5 bis 10 sinkt während der Einwirkungszeit bis auf 6,0 bis 7,5 ab. Die Einwirkungszeit ist abhängig von der Stärke der gewünschten Krause und von der Behandlungstemperatur. Nach der Behandlung werden die Haare oxidativ nachbehandelt und getrocknet.

In einer weiteren bevorzugten Ausführungsform der erfindungsgemäßen Verfahrens wäscht man das Haar zunächst mit einem Shampoo und spült danach mit Wasser. Anschließend wird das handtuchtrockene Haar in einzelne Strähnen aufgeteilt und auf Wickler mit einem Durchmesser von 5 bis 30 Millimetem, bevorzugt 5 bis 15 Millimeter, gewickelt. Nachdem 10 Sekunden bis 3 Minuten vor der Anwendung die alkalisch hydrolisierbare Verbindung der Komponente B mit dem die haarkeratinreduzierende Mercaptoverbindung und die Puffersubstanzen Ammoniumhydrogencarbonat, Ammoniumcarbonat und/oder Ammoniumcarbamat enthaltenden Komponente A vermischt wurde, wird das Haar mit einer für die Haarverformung ausreichenden Menge, vorzugsweise 50 bis 120 Gramm, des gebrauchsfertigen Verformungsmittels behandelt.

Nach einer für die dauerhafte Verformung des Haares ausreichenden Einwirkungszeit, welche je nach Haarbeschaffenheit, der Verformungswirksamkeit des Verformungsmittels sowie in Abhängigkeit von der Anwendungstemperatur 5 bis 30 Minuten (10 bis 30 Minuten ohne Wärmeeinwirkung oder 5 bis 20 Minuten mit Wärmeeinwirkung) beträgt, wird das Haar mit Wasser gespült und dann oxidativ nachbehandelt ("fixiert"). Das Nachbehandlungsmittel wird, je nach Haarfülle, vorzugsweise in einer Menge von 80 bis 100 Gramm, verwendet.

Für die oxidative Nachbehandlung im aufgewickelten oder abgewickelten Zustand kann jedes beliebige, für eine derartige Behandlung geeignete Nachbehandlungsmittel verwendet werden. Beispiele für in solchen Nachbehandlungsmitteln verwendbare Oxidationsmittel sind Kalium- und Natriumbromat, Natriumperborat, Hamstoff- und Wasserstoffperoxid. Die Konzentration des Oxidationsmittels ist in Abhängigkeit von der Anwendungszeit (in der Regel 5 bis 15 Minuten) und der Anwendungstemperatur unterschiedlich. Normalerweise liegt das Oxidationsmittel in dem gebrauchsfertigen wäßrigen Nachbehandlungsmittel in einer Konzentration von 0,5 bis 10 Gew.-% vor. Das Mittel für die oxidative Nachbehandlung kann selbstverständlich weitere Stoffe, wie z. B. Netzmittel, Pflegestoffe wie kationaktive Polymere, schwache Säuren, Puffersubstanzen oder Peroxidstabilisatoren, enthalten und in Form einer wäßrigen Lösung, einer Emulsion sowie in verdickter Form auf wäßriger Basis, insbesondere als Creme, Gel oder Paste, vorliegen. Diese üblichen Zusätze können insbesondere in einer Menge von 0,1 bis 10 Gew.-% in dem Nachbehandlungsmittel enthalten sein.

Anschließend werden die Wickler entfernt. Falls erforderlich, kann das abgewickelte Haar nun nochmals oxidativ nachbehandelt werden. Dann wird das Haar mit Wasser gespült, gegebenenfalls zur Wasserwelle gelegt und schließlich getrocknet.

Das Verfahren bewirkt eine elastische, dauerhafte und gleichmäßige Umformung vom Haaransatz bis zur Haarspitze, ohne allergische oder sensibilisierende Reaktionen hervorzurufen unter weitgehender Schonung der Haarstruktur vor Schädigung.

### Beispiele

### Beispiel 1 Zweikomponenten-Haarverförmungsmittel

| | | |
|---|---|---|
| Komponente A: | 14,5 g | Ammoniumthioglykolat, 70%ige Wäßrige Lösung |
| | 2,0 g | Thiomilchsäure |
| | 2,0 g | Ammoniumcarbonat |
| | 0,7 g | Vinylpyrrolidon/Methacrylamidopropyltrimethylammoniumchlorid Copolymer (CTFA:Polyquatemium-28) |
| | 1, 0 g | hydriertes, ethoxyliertes Ricinusöl (CTFA: PEG-40 Hydrogenated Castor Oil) |
| | 0,5 g | Parfümöl |
| | bis pH 9,4 | Ammoniak |
| | ad 100,0 g | Wasser |
| Komponente B: | 100,0 g | 2-Acetoxybenzoesäure |

Vor Gebrauch werden 72 g der Komponente A und 8 g der Komponente B miteinander vermischt. Der anfängliche pH-Wert des gebrauchsfertigen Verformungsmittels von 9,4 wird während der Einwirkungszeit durch Verseifung der 2-Acetoxybenzoesäure allmählich um 2,1 Einheiten nach 7,3 verringert:

Das gewaschene und frottierte Haar wird auf Wickler mit einem Durchmesser von 6 Millimetern gewickelt und dann mit dem 1 Minute vor der Anwendung hergestellten, vorstehend beschriebenen. Haarverformungsmittel gleichmäßig befeuchtet. Nach der Einwirkungszeit von 20 Minuten bei Raumtemperatur wird das Haar gründlich mit Wasser gespült und dann mit 80 Gramm einer dreiprozentigen wäßrigen Wasserstoffperoxidlosung oxidativ nachbehandelt. Nach Entfernung der Wickler werden die Haare emeut mit Wasser gespült, zur Wasserwelle gelegt und anschließend getrocknet.

### Beispiel 2 Zweikomponenten-Haarverfocmungsmittel

| | | |
|---|---|---|
| Komponente A: | 11,2 g | Ammoniumthioglykolat, 70%ige wäßrige Lösung |
| | 5,3 g | Ammoniumthiolactat, 50%ige wäßrige Lösung |
| | 2,0 g | Ammoniumhydrogencarbonat |
| | 1,7 g | Polyoxyethylen (4) Laurylether (CTFA: Laureth-4) |
| | 1,5 g | Vinylpyrrolidon/Methacrylamidopropyl-trimethylammoniumchlorid Copolymer (CTFA: Polyquatemium-28) |
| | 0,8 g | Parfümöl |
| | bis pH 8,3 | Ammoniak |
| | ad 100,0 g | Wasser |
| Komponente B: | 100,0 g | Galactonsäure-1,4-lacton |

Vor Gebrauch werden 72,0 g der Komponente A und 8,0 g der Komponente B miteinander vermischt. Die Anwendung des Haarverformungsmittels erfolgt analog Beispiel 1. Der anfängliche pH-Wert des gebrauchsfertigen Verformungsmittels von 8,3 wird während der Einwirkungszeit durch Verseifung des Galactonsäure-1,4-lactons allmählich um 1,6 Einheiten nach 6,7 verringert.

### Beispiel 3 Zweikomponenten-Haarverformungsmittel

| | | |
|---|---|---|
| Komponente A: | 14,8 g | Ammoniumthioglykolat, 70%ige wäßrige Lösung |
| | 2,0 g | Ammoniumhydrogencarbonat |
| | 0,6 g | Vinylpyrrolidon / Methylvinylimidazolium-chlorid Copolymer (CTFA: Polyquaternium-16) |
| | 1,9 g | Kokosfettalkholpolyoxyethylenether (10 EO) (CTFA: Coceth-10) |
| | 0,5 g | Parfümöl |
| | bis pH 8,2 | Ammoniak |
| | ad 100,0 g | Wasser |
| Komponente B: | 100,0 g | Ribansäure-1,4-lacton |

Vor Gebrauch werden 72,0 g der Komponente A und 8,0 g der Komponente B miteinander vermischt. Die Anwendung des Haarverformungsmittels erfolgt analog Beispiel 1. Der anfängliche pH-Wert des gebrauchsfertigen Verformungsmittels von 8,2 wird während der Einwirkungszeit durch Verseifung des Ribonsäure-1,4-lactons allmählich um 1,6 Einheiten auf 6,6 verringert.

### Beispiel 4 Zweikomponenten-Haarverformungsmittel

| | | |
|---|---|---|
| Komponente A: | 11,2 g | Ammoniumthioglykolat, 70%ige wäßrige Lösung |
| | 6,0 g | Ammoniumthiolactat, 50%ige wäßrige Lösung |
| | 2,0 g | Ammoniumhydrogencarbonat |
| | 0,7 g | Vinylpyrrolidon / N,N-Dimethylaminoethylmethacrylat Copolymer (CTFA: Polyquaternium-11) |
| | 2,0 g | Polyoxyethylen (4) Laurylether (CTFA: Laureth-4) |
| | 0,8 g | Parfümöl |
| | bis pH 8,2 | Ammoniak |
| | ad 100,0 g | Wasser |
| Komponente B: | 100,0 g | Mannonsäure-1,4-lacton |

Vor Gebrauch werden 72,0 g der Komponente A und 8,0 g der Komponente B miteinander vermischt. Die Anwendung des Haarverformungsmittels erfolgt analog Beispiel 1. Der anfängliche pH-Wert des gebrauchsfertigen Verformungsmittels von 8,2 wird während der Einwirkungszeit durch Verseifung des Mannonsäure-1,4-lacton allmählich um 1,3 Einheiten auf 6,9 verringert.

### Beispiel 5 Zweikomponenten-Haarverformungsmittel

| | | |
|---|---|---|
| Komponente A: | 11,2 g | Ammoniumthioglykolat, 70%ige wäßrige Lösung |
| | 5,3 g | Ammoniumthiolactat, 50%ige wäßrige Lösung |
| | 2,0 g | Ammoniumhydrogencarbonat |
| | 0,4 g | Vinylpyrrolidon / N,N-Dimethylaminoethylmethacrylat Copolymer (CTFA: Potyquatemium-11) |
| | 2,2g | Polyoxyethylen (20) Sorbitan Monopalmitat (CTFA: Polysorbat 40) |
| | 0,8 g | Parfümöl |
| | bis pH 8,2 | Ammoniak |
| | ad 100,0 g | Wasser |
| Komponente B: | 100,0 g | Gulonsäure-1,4-lacton |

Vor Gebrauch werden 72,0 g der Komponente A und 8,0 g der Komponente B miteinander vermischt. Die Anwendung des Haarverformungsmittels erfolgt analog Beispiel 1. Der anfängliche pH-Wert des gebrauchsfertigen Verformungsmittels von 8,2 wird während der Einwirkungszeit durch Verseifung des Gulonsäure-1,4-lactons allmählich um 1,4 Einheiten auf 6,8 verringert.

### Beispiel 6 Zweikomponenten-Haarverformungsmittel

| | | |
|---|---|---|
| Komponente A: | 12,2 g | Ammoniumthioglykolat, 70%ige wäßrige Lösung |
| | 3,3 g | Cystein |
| | 2,0 g | Ammoniumhydrogencarbonat |
| | 0,7 g | Vinylpyrrolidon / Methacrylamidopropyltrimethylammoniumchlorid Copolymer (CTFA: Polyquatemium-28) |
| | 1,5 g | Polyoxyethylen (4) Laurylether (CTFA: Laureth-4) |
| | 0,8 g | Parfümöl |
| | bis pH 8,5 | Ammoniak |
| | ad 100,0 g | Wasser |
| Komponente B: | 100,0 g | Methylvinylether/Maleinsäureanhydrid Copolymer (CTFA: PVM/MA Copolymer) |

Vor Gebrauch werden 78,4 g der Komponente A und 1,6 g der Komponente B miteinander vermischt. Die Anwendung des Haarverformungsmittels erfolgt analog Beispiel 1. Der anfängliche pH-Wert des gebrauchsfertigen Verformungsmittels von 8,5 wird während der Einwirkungszeit durch Hydrolyse des Methylvinylether/Maleinsäureanhydrid Copolymers allmählich um 1,3 Einheiten auf 7,2 verringert.

### Beispiel 7 Zweikomponenten-Haarverformungsmittel

| | | |
|---|---|---|
| Komponente A: | 18,4 g | Ammoniumhioglykolat, 70%ige wäßrige Lösung |
| | 8,1 g | Ammoniumthiolactat, 70%ige wäßrige Lösung |
| | 3,9 g | Thiomilchsäure, 99%ige wäßrige Lösung |
| | 4,0 g | Ammoniumhydrogencarbonat |
| | 0,7 g | Dimethyldiallylammoniumchlorid-Homopolymer (CTFA: Polyquaternium-6) |
| | 1,5 g | hydriertes, ethoxyliertes Ricinusöl (CTFA: PEG-40 Hydrogenated Castor Oil) |
| | 0,8 g | Parfümöl |
| | bis pH 8,5 | Ammoniak |
| | ad 100,0 g | Wasser |
| Komponente B: | 100,0 g | Glucuronsäure-6,3-lacton |

Vor Gebrauch werden 74,0 g der Komponente A und 6,0 g der Komponente B miteinander vermischt. Die Anwendung des Haarverformungsmittets erfolgt analog Beispiel 1. Der anfängliche pH-Wert des gebrauchsfertigen Verformungsmittels von 8,5 wird während der Einwirkungszeit durch Verseifung des Glucuronsäure-6,3-lactons allmählich um 1,2 Einheiten auf 7,3 verringert.

### Beispiel 8 Zweikomponenten-Haarverformungsmittel

| | | |
|---|---|---|
| Komponente A: | 18,4 g | Ammoniumhioglykolat, 70%ige wäßrige Lösung |
| | 8,1g | Ammoniumthiolactat, 70%ige wäßrige Lösung |
| | 3,9 g | Thiomilchsäure, 99%ige wäßrige Lösung |
| | 4,0 g | Ammoniumhydrogencarbonat |
| | 0,3 g | Dimethyldiallylammoniumchlorid-Homopolymer (CTFA: Polyquatemium-6) |
| | 2,0 g | Glycerin-polyethylenglycolricinoleat (CTFA: PEG-35 Castor Oil) |
| | 0,8 g | Parfümöl |
| | bis pH 8,5 | Ammoniak |
| | ad 100,0 g | Wasser |
| Komponente B: | 100,0 g | Gluconsäure-1,5-lacton |

Vor Gebrauch werden 75,2 g der Komponente A und 4,8 g der Komponente B miteinander vermischt. Die Anwendung des Haarverformungsmittels erfolgt analog Beispiel 1. Der anfängliche pH-Wert des gebrauchsfertigen Verformungsmittels von 8,5 wird während der Einwirkungszeit durch Verseifung des Gluconsäure-1,5-lactons allmählich um 1 Einheit auf 7,5 verringert.

### Beispiel 9 Zweikomponenten-Haarverformungsmittel

| | | |
|---|---|---|
| Komponente A: | 13,3 g | Ammoniumthioglykolat, 70%ige wäßrige Lösung |
| | 2,0 g | Ammoniumhydrogencarbonat |
| | 0,9 g | Vinylpyrrolidon / Methylvinylimidazolium-chlorid Copolymer (CTFA: Polyquaternium-16) |
| | 1,5 g | Kokosfettalkhofpolyoxyethylenether-(10 EO) (CTFA: Coceth-10) |
| | 0,8 g | Parfümöl |
| | bis pH 8,5 | Ammoniak |
| | ad 100,0 g | Wasser |
| Komponente B: | 87,0 g | Glycerin |
| | 13,0 g | Gluconsäure-1,5-lacton |

Vor Gebrauch werden 61,5 g der Komponente A und 18,5 g der Komponente B miteinander vermischt. Die Anwendung des Haarverformungsmittels erfolgt analog Beispiel 1. Der anfängliche pH-Wert des gebrauchsfertigen Verformungsmittels von 8,5 wird während der Einwirkungszeit durch Verseifung des Gluconsäure-1,5-lactons allmählich um 1,2 Einheiten auf 7,3 verringert.

### Beispiel 10 Zweikomponenten-Haarverformungsmittel

| | | |
|---|---|---|
| Komponente A: | 11,9 g | Thioglykolsäure |
| | 1,0 g | Ammoniumhydrogencarbonat |
| | 0,7 g | Vinylpyrrolidon / Methacrylamidopropyltrimethylammoniumchlorid Copolymer (CTFA: Polyquatemium-28) |
| | 1,5 g | Polyoxyethylen (4) Laurylether (CTFA: Laureth-4) |
| | 0,8 g | Parfümöl |
| | bis pH 8,5 | Ammoniak |
| | ad 100,0 g | Wasser |
| Komponente B: | 50,0 g | Glucuronsäure-6,3-lacton |
| | 50,0 g | Gluconsäure-1,5-lacton |

Vor Gebrauch werden 79,2 g der Komponente A und 0,8 g der Komponente B miteinander vermischt. Die Anwendung des Haarverformungsmittels erfolgt analog Beispiel 1. Der anfängliche pH-Wert des gebrauchsfertigen Verformungsmittels von 8,5 wird während der Einwirkungszeit durch Verseifung der Ester allmählich um 0,6 Einheiten auf 7,9 verringert.

## Patentansprüche

1. Verfahren zur dauerhaften Verformung von Haaren, bei dem man unmittelbar vor der Anwendung einem alkalischen Dauerverformungsmittel mit einem Gehalt an einem keratinreduzierenden Wirkstoff und einem anfänglichen pH-Wert von 7,5 bis 10 eine alkalisch hydrolisierbare Verbindung zusetzt, das Haar bevor und/oder nachdem man es in der gewünschten Form festhält, mit dem gebrauchsfertigen Mittel behandelt, nach der Einwirkungszeit das Haar mit Wasser spült, oxidativ nachbehandelt, emeut mit Wasser spült, gegebenenfalls zur Wasserwelle legt, und sodann trocknet, **dadurch gekenn-zeichnet,daß** die alkalisch hydrolisierbare Verbindung so ausgewählt wird, daß der anfängliche pH-Wert ohne den Zusatz eines esterspaltenden Enzyms innerhalb von 5 bis 30 Minuten um mindestens 0,5 Einheiten herabgesetzt wird, und die alkalisch hydrolisierbare Verbindung ausgewählt ist aus den Gruppen: Lacton einer Onsäure oder einer Uronsäure, Acylsalicylsäure mit 1 bis 5 Kohlenstoffatomen in der Acylgruppe, und polymeres Anhydrid.

2. Verfahren nach Anspruch 1, **dadurch gekennzeichnet, daß** der anfängliche pH-Wert innerhalb von 7 bis 20 Minuten um mindestens eine Einheit auf einen pH-Wert zwischen 6,5 bis 7,3 herabgesetzt wird.

3. Verfahren nach einem der Ansprüche 1 oder 2, **dadurch gekennzeichnet, daß** der pH-Wert des Dauerwellmittels anfänglich 8 bis 9,5 beträgt und innerhalb von 10 bis 30 Minuten um 0,8 bis 2,0 Einheiten abgesenkt wird.

4. Verfahren nach Anspruch 3, **dadurch gekennzeichnet, daß** das Lacton der On- oder Uronsäure das Lacton einer Aldohexose ist.

5. Verfahren nach Anspruch 4, **dadurch gekennzeichnet, daß** das Lacton der On- oder Uronsäure ausgewählt ist aus Gluconsäure-1,5-lacton und Glucuronsäure-6,3-lacton.

6. Verfahren nach einem der Ansprüche 1 bis 5, **dadurch gekennzeichnet, daß** die alkalisch spaltbaren organischen Verbindung gelöst in einem nicht wäßrigen Lösungsmittel vorliegt.

7. Verfahren nach Ansprüche 6, **dadurch gekennzeichnet, daß** das nicht wäßrige Lösungsmittel ein mehrwertiger Alkohol ist.

8. Verfahren nach Anspruch 7, **dadurch gekennzeichnet, daß** der mehrwertige Alkohol Glycerin ist.

9. Verfahren nach einem der Ansprüche 1 bis 8, **dadurch gekennzeichnet, daß** man dem alkalischen Dauerverformungsmittel unmittelbar vor der Anwendung zusätzlich einen Puffer zusetzt, dessen Wirkungsbereich bei pH 6,8 bis 7,5 liegt.

10. Verfahren nach Anspruch 9, **dadurch gekennzeichnet, daß** der Puffer Ammoniumhydrogencarbonat ist.

## Claims

1. Method for permanently shaping hair, in which, directly prior to use, an alkaline-hydrolysable compound is added to an alkaline permanent shaping agent with a content of a keratin-reducing active ingredient and an initial pH of from 7.5 to 10, the hair is before or after held in the desired shape, treated with the ready-to-use agent, after the contact time the hair is rinsed with water, oxidatively after-treated, rinsed with water again, optionally set and then dried, **characterized in that** the alkaline-hydrolysable compound is chosen so that the initial pH is reduced by at least 0.5 units without adding an ester-cleaving enzyme over the course of from 5 to 30 minutes and the alkaline-hydrolysable compound is chosen from the groups: lactone of an aldonic acid or of a uronic acid, acylsalicylic acid having 1 to 5 carbon atoms in the acyl group, and polymeric anhydride.

2. Method according to Claim 1, **characterized in that** the initial pH is reduced by at least one unit to a pH of between 6.5 to 7.3 over the course of from 7 to 20 minutes.

3. Method according to one of Claims 1 or 2,
**characterized in that** the pH of the permanent waving agent is initially 8 to 9.5 and is reduced by from 0.8 to 2.0 units over the course of from 10 to 30 minutes.

4. Method according to Claim 3, **characterized in that** the lactone of aldonic acid or uronic acid is the lactone of an aldohexose.

5. Method according to Claim 4, **characterized in that** the lactone of the aldonic acid or uronic acid is chosen from glucono-1,5-lactone and glucurono-6,3-lactone.

6. Method according to one of Claims 1 to 5, **characterized in that** the alkaline-cleavable organic compound is present in dissolved form in a nonaqueous solvent.

7. Method according to Claim 6, **characterized in that** the nonaqueous solvent is a polyhydric alcohol.

8. Method according to Claim 7, **characterized in that** the polyhydric alcohol is glycerol.

9. Method according to one of Claims 1 to 8, **characterized in that**, directly prior to use, a buffer whose effect range is at pH 6.8 to 7.5 is additionally added to the alkaline permanent shaping agent.

10. Method according to Claim 9, **characterized in that** the buffer is ammonium hydrogencarbonate.

## Revendications

1. Procédé pour la mise en forme permanente des cheveux, dans lequel on ajoute, immédiatement avant l'emploi, un composé apte à l'hydrolyse alcaline, à une composition alcaline de mise en forme permanente ayant une teneur en une substance active réduisant la kératine et un pH initial de 7,5 à 10, on traite les cheveux par la composition prête à l'emploi, avant et/ou après leur maintien en la forme désirée, après le temps d'action on rince les cheveux à l'eau, on les soumet à un après-traitement oxydant, on les rince de nouveau à l'eau, éventuellement on les met en plis et ensuite on les sèche, **caractérisé en ce que** le composé apte à l'hydrolyse alcaline est choisi de manière que le pH initial, sans l'addition d'une enzyme hydrolysant les esters, est abaissé d'au moins 0,5 unité en l'espace de 5 à 30 minutes, et le composé apte à l'hydrolyse alcaline est choisi dans les groupes : lactone d'un acide onique ou d'un acide uronique, acide acylsalicylique ayant de 1 à 5 atomes de carbone dans le groupe acyle et anhydride polymère.

2. Procédé selon la revendication 1, **caractérisé en ce que** le pH initial est abaissé d'au moins une unité, jusqu'à un pH compris entre 6,5 et 7,3, en l'espace de 7 à 20 minutes.

3. Procédé selon la revendication 1 ou 2, **caractérisé en ce que** le pH de la composition d'ondulation permanente va initialement de 8 à 9,5 et est abaissé de 0,8 à 2,0 unités en l'espace de 10 à 30 minutes.

4. Procédé selon la revendication 3, **caractérisé en ce que** la lactone de l'acide onique ou uronique est la lactone d'un aldohexose.

5. Procédé selon la revendication 4, **caractérisé en ce que** la lactone de l'acide onique ou uronique est choisie parmi la glucono-1,5-lactone et la glucurono-6,3-lactone.

6. Procédé selon l'une quelconque des revendications 1 à 5, **caractérisé en ce que** le composé organique apte à la coupure alcaline se trouve en solution dans un solvant non aqueux.

7. Procédé selon la revendication 6, **caractérisé en ce que** le solvant non aqueux est un alcool polyhydrique.

8. Procédé selon la revendication 7, **caractérisé en ce que** l'alcool polyhydrique est le glycérol.

9. Procédé selon l'une quelconque des revendications 1 à 8, **caractérisé en ce que**, immédiatement avant l'emploi, on ajoute en outre à la composition alcaline de mise en forme permanente un tampon dont l'intervalle d'action va de pH 6,8 à 7,5.

10. Procédé selon la revendication 9, **caractérisé en ce que** le tampon est l'hydrogénocarbonate d'ammonium.
